# FASCICULE DE BREVET EUROPEEN

(11) **EP 0 649 320 B1**
(45) Date de publication et mention de la délivrance du brevet: **11.08.1999**
(21) Numéro de dépôt: 93909015.5
(22) Date de dépôt: 09.04.1993
(51) Int. Cl.: A61M 5/50

(54) **SERINGUE UNI INJECTION**
EINWEGSPRITZE
SINGLE-USE SYRINGE

(30) Priorité: 10.04.1992 FR 9204384
(43) Date de publication de la demande: 26.04.1995
(73) Titulaire: VOUNATSOS, Constantin, F-78490 Galluis (FR)
(72) Inventeur: VOUNATSOS, Constantin, F-78490 Galluis (FR)
(74) Mandataire: Armengaud Ainé, Alain
(86) Numéro de dépôt international: FR9300362
(87) Numéro de publication internationale: WO9320873

(56) Documents cités:
- EP-A- 0 392 228
- EP-A- 0 409 134
- FR-A- 2 653 668
- GB-A- 2 015 883
- GB-A- 2 202 747
- US-A- 4 731 068
- US-A- 4 863 427
- US-A- 4 932 941

## Description

La présente invention concerne un dispositif pour rendre inutilisable une seringue après une première injection. Les seringues telles que fabriquées actuellement peuvent se réutiliser après une première injection et sont donc des vecteurs de transmission et de propagation de diverses maladies.

On connaît par le document GB-A-2 015 883 ou par US-A-4 932 941 (qui correspond au préambule de la revendication 1), ou par EP-A-0 409 134, ou par GB-A-2 202 747, ou par US-A-4 863 427, des dispositifs permettant de séparer la tête d'un piston du corps d'une seringue après un premier usage.

La seringue selon l'invention propose un assemblage amélioré entre ces deux pièces.

A cet effet, la seringue inutilisable après une première injection, comprend un corps dans lequel se translate un corps de piston coopérant à une tête de piston, ledit corps de seringue comprenant une surépaisseur de matière formant bague dans le cylindre intérieur du corps à une distance proche du point de sortie du liquide à injecter et une seconde surépaisseur de matière formant bague dans le cylindre intérieur du corps à une position proche de la sortie du piston de la seringue, ayant entre la tête du piston et le corps du piston, un dispositif d'assemblage entre ces deux pièces composé d'une pièce intégrale à la tête de piston de diamètre substantiellement réduit, celle-ci n'étant pas en contact avec la paroi du corps de la seringue, cette partie intégrale de la tête de piston constituant la connexion mâle/femelle, cette connexion mâle/femelle comportant comme partie mâle, une pièce tronçonnée avec une face antérieure fendue faisant partie intégrante du corps de piston, alors que la partie correspondante femelle comprend une cavité symétrique à cette partie mâle vais opposée de façon à ce que la partie mâle puisse facilement être retirée de la partie femelle mais ne puisse pas être réintroduite, cette partie femelle étant partie intégrante de la tête, le diamètre du corps de piston correspondant substantiellement au diamètre interne de la seringue, la surépaisseur faisant bague placée à une distance très proche du point de sortie du liquide à injecter coopérant avec le dispositif d'assemblage et provoquant l'abandon définitif et irréversible de la tête de piston dans le corps de la seringue lors du recul du corps de piston, la surépaisseur formant la bague garantissant le non retrait du corps de piston du corps de la seringue afin de détruire le dispositif d'assemblage mâle/femelle.

Selon des modes particuliers de réalisation :
- Le corps de la seringue peut comporter dans la partie intérieure qui reçoit le piston, deux surépaisseurs qui provoqueront à ces endroits des changements de diamètres intérieurs.
   L'une se situera à une distance proche du point de sortie du liquide à injecter, l'autre proche de l'entrée du corps de la seringue.
   La première surépaisseur aura pour but de servir de butée de non retour pour retenir la tête du piston et provoquer le désassemblage de la tête du piston du corps du piston, quand on ramène celui-ci en arrière, après une première injection.
   La seconde surépaisseur empêche de retirer du corps de la seringue, l'ensemble tête de piston et corps de piston avant une première injection, pour violer le dispositif de désassemblage.
- Le piston peut être constitué d'un corps et d'une tête.

Ces deux parties seront assemblées par un système comportant une cavité cylindrique fendue côté tête de piston, et un cylindre tronconné côté corps du piston. La partie tronconnée du cylindre empêche de réengager le cylindre dans la cavité cylindrique fendue de la tête du piston, quand l'assemblage a été désassemblé une première fois.

En variante, le système d'assemblage peut être constitué d'une cavité trapézoïdale fendue côté tête de piston, et d'un trapèze mâle côté corps de piston.

Les dessins annexés illustrent l'invention :

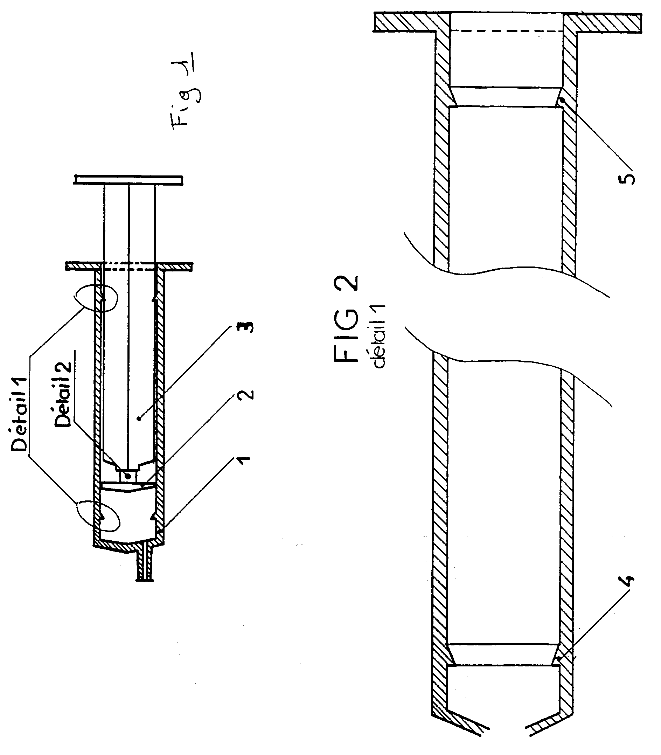

La figure 1 représente en coupe, le dispositif selon l'invention.
La figure 2 représente en coupe le détail du changement de diamètre du corps de la seringue selon l'invention.

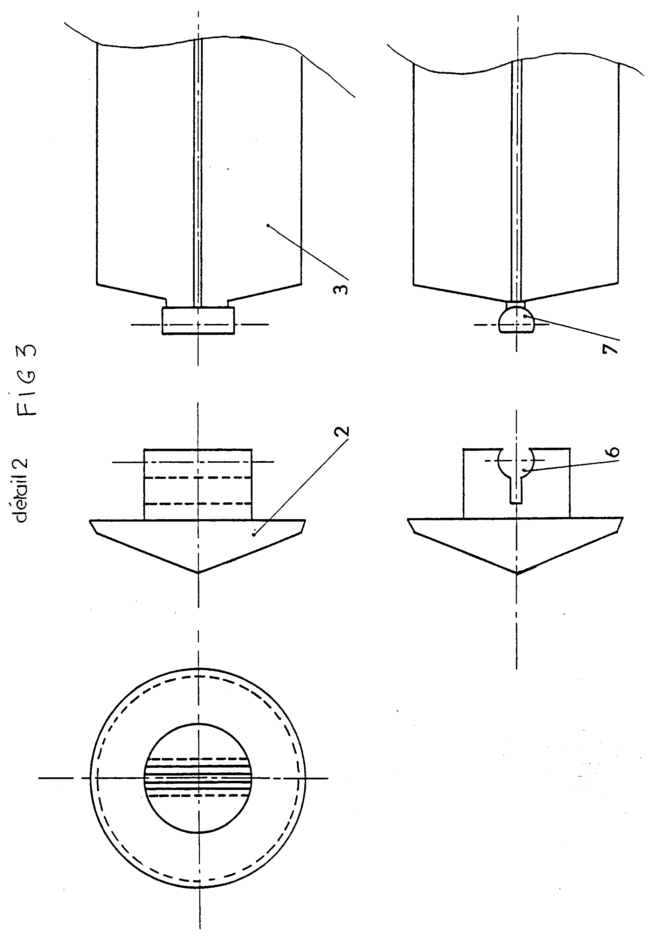

La figure 3 représente l'assemblage entre la tête de piston et le corps du piston suivant l'invention.

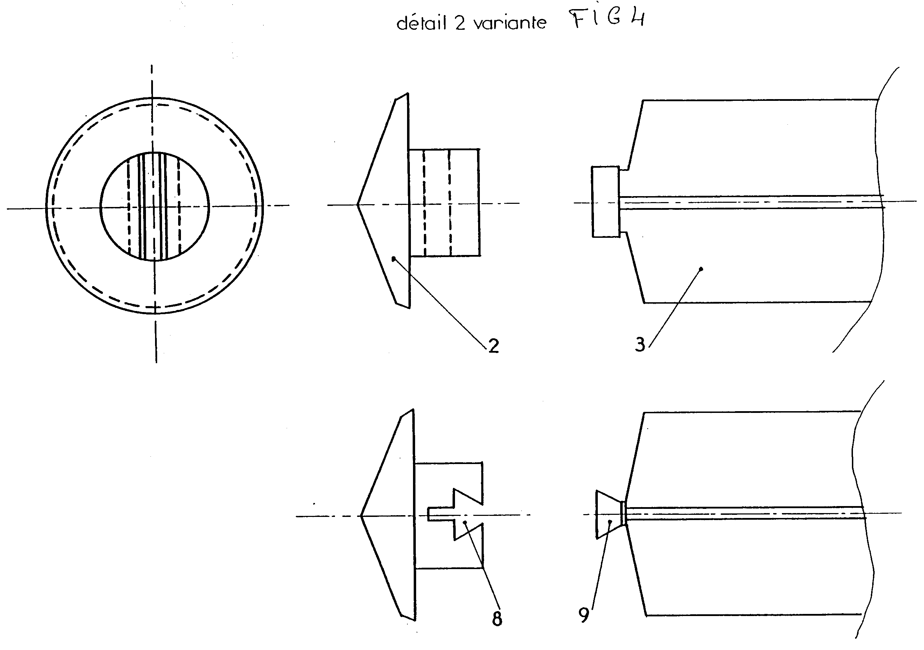

La figure 4 représente la variante de l'assemblage entre la tête de piston et le corps du piston suivant l'invention.

En référence à ces dessins, le dispositif comporte un corps de seringue (1) conçu de façon à créer deux bagues en surépaisseur (4) et (5), servant de butée de non retour dans le cylindre intérieur.

La tête de piston (2) conçue pour s'ajuster de façon parfaite avec le corps de seringue, comporte à sa base un assemblage composé d'une cavité femelle cylindrique (6) ou trapézoïdale (8) (en variante) fendue pour faciliter l'élasticité.

Le piston (2) de diamètre extérieur légèrement inférieur au diamètre intérieur du corps de la seringue, se termine par une pièce mâle, cylindrique (7) ou trapézoïdale en variante (9).

Le dispositif, selon l'invention, est destiné à rendre la seringue inutilisable après une première injection. Sous l'effet d'une tentative de retrait de piston, la résistance de la butée étant supérieure à la résistance de l'assemblage, les deux pièces tête de piston et corps du piston se désassemblent et condamnent définitivement la réutilisation de la seringue.

## Revendications

1. Seringue inutilisable après une première injection, comprenant un corps (1) dans lequel se translate un corps de piston (3) coopérant à une tête de piston (2), ledit corps de seringue (1) comprenant une surépaisseur de matière formant bague (4) dans le cylindre intérieur du corps (1) à une distance proche du point de sortie du liquide à injecter et une seconde surépaisseur de matière formant bague (5) dans le cylindre intérieur du corps (1) à une position proche de la sortie du piston de la seringue, un dispositif d'assemblage mâle/femelle étant disposé entre la tête du piston (2) et le corps du piston (3), le diamètre du corps de piston (3) correspondant substantiellement au diamètre interne de la seringue, la surépaisseur faisant bague (4) placée à une distance très proche du point de sortie du liquide à injecter coopérant avec le dispositif d'assemblage et provoquant l'abandon définitif et irréversible de la tête de piston (2) dans le corps (1) de la seringue lors du recul du corps de piston (3), la surépaisseur formant la bague (5) garantissant le non retrait du corps de piston (3) du corps de la seringue (1) afin de détruire le dispositif d'assemblage mâle/femelle, caractérisée en ce que ledit dispositif d'assemblage est composé d'une pièce intégrale à la tête de piston de diamètre substantiellement réduit, celle-ci n'étant pas en contact avec la paroi du corps (1) de la seringue, cette partie intégrale de la tête de piston (2) constituant la connexion mâle/femelle, cette connexion mâle/femelle comportant comme partie mâle, une pièce tronçonnée avec une face antérieure découpée faisant partie intégrante du corps de piston (3), alors que la partie correspondante femelle comprend une cavité symétrique à cette partie mâle mais opposée de façon à ce que la partie mâle puisse facilement être retirée de la partie femelle mais ne puisse pas être réintroduite, cette partie femelle étant partie intégrante de la tête (2).

2. Seringue selon la revendication 1, caractérisée en ce que ce que le corps (1) présente une surépaisseur formant butée de non retour (4) et une surépaisseur formant butée de sécurité (5).

3. Seringue selon la revendication 1, caractérisée en ce que le dispositif d'assemblage comporte, d'une part à la tête du piston (2), au niveau de sa base, un assemblage femelle composé d'une cavité cylindrique fendue (6), la fente servant à provoquer l'élasticité du logement femelle, et d'autre part, au niveau de l'extrémité du piston (3), un cylindre tronçonné (7) coopérant au niveau de la cavité cylindrique (6), la partie tronçonnée empêchant le réassemblage après un premier désassemblage.

4. Seringue selon la revendication 1, caractérisée en ce que le dispositif d'assemblage comporte, d'une part à la tête du piston (2), au niveau de sa base, un assemblage femelle de forme trapézoïdale fendue (8), la fente servant à provoquer l'élasticité du logement femelle, et d'autre part au niveau de l'extrémité du piston (3), une pièce mâle trapézoïdale (9).

## Claims

1. Syringe which is unusable after a first injection, comprising a body (1) in which a piston body (3) moves up and down, cooperating with a piston head (2), said syringe body (1) comprising an excess thickness of material forming a ring (4) in the inner cylinder of the body (1) at a distance close to the outlet point for the liquid to be injected and a second excess thickness of material forming a ring (5) in the inner cylinder of the body (1) at a position close to the outlet of the piston from the syringe, a male/female assembly device being disposed between the head of the piston (2) and the body of the piston (3), the diameter of the piston body (3) corresponding substantially to the inner diameter of the syringe, the excess thickness forming the ring (4) placed at a distance very close to the outlet point of the liquid to be injected cooperating with the assembly device and causing the definitive and irreversible abandonment of the piston head (2) in the body (1) of the syringe at the time of the return of the piston body (3), the excess thickness forming the ring (5) guaranteeing the non-withdrawal of the piston body (3) from the syringe body (1) in order to destroy the male/female assembly device, characterised in that said assembly device is composed of a member integral with the piston head of substantially reduced diameter, the latter not being in contact with the wall of the syringe body (1), this part integral with the piston head (2) constituting the male/female connection, this male/female connection comprising as the male part, a truncated member with a cut-out front face forming an integral part of the piston body (3), whereas the corresponding female part comprises a cavity symmetrical with this male part but opposed so that the male part may easily be withdrawn from the female part but cannot be re-introduced, this female part being an integral part of the head (2).

2. Syringe according to Claim 1, characterised in that the body (1) has an excess thickness forming a non-return stop (4) and an excess thickness forming a safety stop (5).

3. Syringe according to Claim 1, characterised in that the assembly device comprises, on the one hand on the head of the piston (2), at the level of its base, a female assembly composed of a split cylindrical cavity (6), the slot serving to cause the elasticity of the female housing, and on the other hand, at the level of the end of the piston (3), a truncated cylinder (7) cooperating at the level of the cylindrical cavity (6), the truncated part preventng re-assembly after a first disassembly.

4. Syringe according to Claim 1, characterised in that the assembly device comprises, on the one hand on the head of the piston (2), at the level of its base, a female assembly of split trapezoidal shape (8), the slot serving to cause the elasticity of the female housing, and on the other hand, at the level of the end of the piston (3), a trapezoidal male member (9).

## Patentansprüche

1. Spritze, die nach einer ersten Injektion nicht wieder verwendet werden kann mit einem Körper (1), in dem ein Kolbenkörper (3) verschiebbar gehalten ist, der mit einem Kolbenkopf (2) zusammenwirkt, wobei der Spritzenkörper (1) eine Überdicke aufweist, die im Innenzylinder des Körpers (1) in geringem Abstand vom Austrittspunkt der zu injizierenden Flüssigkeit einen Ring (4) bildet, und eine zweite Überdicke aufweist, die im Innenzylinder des Körpers (1) in geringem Abstand vom Ausgang des Spritzenkolbens einen Ring (5) bildet, und mit einer Stecker/Hülsen-Verbindungsvorrichtung zwischen dem Kolbenkopf (2) und dem Kolbenkörper (3), wobei der Durchmesser des Kolbenkörpers (3) im wesentlichen dem Innendurchmesser der Spritze entspricht, und die den Ring (4) bildende Überdicke, die sehr nahe am Austrittspunkt der zu injizierenden Flüssigkeit angeordnet ist, mit der Verbindungsvorrichtung zusammenwirkt und den endgültigen und irreversiblen Verlust des Kolbenkopfes (2) im Spritzenkörper (1) bewirkt, wenn der Kolbenkörper (3) zurückgezogen wird, und wobei die den Ring (5) bildende Überdicke, indem sie die Stecker/Hülsen-Verbindung zerstört, gewährleistet, daß der Kolbenkörper (3) nicht mit dem Kolbenkopf (2)vom Spritzenkörper (1) abgezogen werden kann, *dadurch gekennzeichnet, daß* die Verbindungsvorrichtung aus einem mit dem Kolbenkopf einstückigen Stück mit wesentlich reduziertem Durchmesser besteht, das die Wand des Spritzenkörpers (1) nicht berührt, wobei dieses mit dem Kolbenkopf (2) einstückige Teil die Stecker /Hülsen-Verbindung bildet, die als Stecker ein abgeschnittenes Stuck mit einer vorderen Schnittfläche aufweist, das mit dem Kolbenkörper (3) einstückig ist, wohingegen die mit dem Kolbenkopf (2) einstückige entsprechende Hülse eine zum Stecker symmetrische, aber entgegengesetzte Aushöhlung aufweist, so daß der Stecker leicht aus der Hülse gezogen, aber nicht wieder eingeschoben werden kann.

2. Spritze nach Anspruch 1, *dadurch gekennzeichnet, daß* der Körper (1) eine Überdicke aufweist, die einen die Rückbewegung des Kolbens verhindernden Anschlag (4) bildet, und eine überdicke, die einen Sicherheits-Anschlag (5) bildet.

3. Spritze nach Anspruch 1, *dadurch gekennzeichnet, daß* die Verbindungsvorrichtung einerseits am Kolbenkopf (2) nahe seiner Basis eine Verbindungshülse aufweist, die aus einer geschlitzten zylindrischen Aushöhlung (6) besteht, wobei der Schlitz die Elastizität der Hülse bewirkt, und andererseits am Ende des Kolbens (3) einen abgeschnittenen Zylinder (7) aufweist, der mit der zylindrischen Aushöhlung (6) zusammenwirkt, wobei der abgeschnittene Teil verhindert, daß die Verbindung nach einer ersten Trennung wieder hergestellt werden kann.

4. Spritze nach Anspruch 1, *dadurch gekennzeichnet, daß* die Verbindungsvorrichtung einerseits am Kolbenkopf (2) an seiner Basis eine trapezförmige, geschlitzte Verbindungshülse (8) aufweist, wobei der Schlitz die Elastizität der Hülse gewährleistet, und andererseits am Ende des Kolbens (3) einen trapezförmigen Verbindungsstecker (9) aufweist.
